# EUROPÄISCHE PATENTSCHRIFT

(11) **EP 4 017 553 B1**
(45) Veröffentlichungstag und Bekanntmachung des Hinweises auf die Patenterteilung: **24.04.2024**
(21) Anmeldenummer: 20760407.5
(22) Anmeldetag: 17.08.2020
(51) Int. Cl.: A61M 1/14

(54) **DIALYSEMASCHINE MIT HALTEVORRICHTUNG ZUM HALTEN UND FIXIEREN ZUMINDEST EINES ELEKTRONISCHEN ENDGERÄTS**
DIALYSIS MACHINE HAVING A HOLDING DEVICE FOR HOLDING AND FIXING AT LEAST ONE ELECTRONIC TERMINAL DEVICE
MACHINE DE DIALYSE POURVUE D'UN DISPOSITIF DE MAINTIEN DESTINÉ À MAINTENIR ET FIXER AU MOINS UN DISPOSITIF TERMINAL ÉLECTRONIQUE

(30) Priorität: 20.08.2019 DE 102019122353
(43) Veröffentlichungstag der Anmeldung: 29.06.2022
(73) Patentinhaber: B. Braun Avitum AG, 34212 Melsungen (DE)
(72) Erfinder: BAUER, Florian, 34212 Melsungen (DE); JANIK, Waldemar, 34212 Melsungen (DE)
(74) Vertreter: Winter, Brandl - Partnerschaft mbB
(86) Internationale Anmeldenummer: PCT/EP2020/073018
(87) Internationale Veröffentlichungsnummer: WO 2021/032692

(56) Entgegenhaltungen:
- DE-A1-102012 020 945
- US-A1- 2011 275 984
- US-A1- 2015 234 363

## Beschreibung

### Technisches Gebiet

Die vorliegende Offenbarung betrifft eine Halterungsvorrichtung zum Halten und Fixieren zumindest eines elektronischen Endgeräts während einer Dialysebehandlung mit einer integrierten Ladevorrichtung.

### Hintergrund der Erfindung

Während einer Dialyse verweilt ein Patient etwa vier bis fünf Stunden auf einer Liege neben der Dialysemaschine. Da ein Patient bis zu dreimal wöchentlich dialysiert werden muss, ergibt dies einen hohen wöchentlichen Zeitaufwand innerhalb des Dialysezentrums. Aufgrund dieser zeitlichen Einschränkungen reagieren Patienten mitunter sehr gereizt, sollte die Therapie länger als gewohnt ablaufen. Außerdem ist der Zeitplan streng getaktet, da beispielsweise Taxis bereits auf die einzelnen Patienten warten, um diese nach Hause zu fahren.

Die wöchentlichen 12 bis 15 Stunden verbringen die meisten Patienten mit Fernsehschauen auf den im Dialysezentrum angebrachten TV-Geräten, Musik hören, Bücher lesen oder schlafen. Ein TV-Gerät ist dabei meist so angebracht, dass drei bis vier Patienten gleichzeitig auf diesem schauen können/müssen und sich dementsprechend über das Fernsehprogramm einig sein müssen.

Die zunehmende Digitalisierung und die damit verbundene weit verbreitete Verfügbarkeit mobiler Endgeräte macht auch vor Patienten von Dialysezentren nicht halt. Auch sie nutzen in großem Umfang mobile, elektronische Geräte, wie beispielsweise Smartphones, E-Book-Reader oder Tablets insbesondere auch, um sich die Zeit während der Dialyse etwas angenehmer zu gestalten.

Gerade die Tendenz, immer mehr Dienste "On-Demand" nutzen zu können und so Serien und Filme sehen zu können, wann immer man möchte, bietet attraktive Möglichkeiten, um die Dialysezeit mit angenehmen Freizeitaktivitäten zu verknüpfen. Daher ist zu erwarten, dass immer mehr Menschen in einem Dialysezentrum ihre Zeit mit der Nutzung mobiler Endgeräte angenehmer gestalten werden.

Größere Medienanwendungen, wie beispielsweise das Streamen/Online-Schauen von Filmen und Serien oder beispielsweise das Spielen von mobilen Spielen, verbrauchen allerdings mitunter viel Energie/Akku und können so einen herkömmlichen Akku eines mobilen Endgeräts schnell entladen. Besonders, da die Fortschritte in der Akkuentwicklung langsamer verlaufen als in den anderen Bereichen/Disziplinen der Unterhaltungselektronik, wird die Möglichkeit, das eigene Gerät schneller wieder aufzuladen, immer wichtiger.

Möchte somit ein Patient sein mobiles Endgerät aufladen, muss dieser sein eigenes Ladegerät in Form einer Power Bank/eines externen Energiespeichermediums mitnehmen. Ein Anschluss eines Ladegeräts an die hauseigenen Steckdosen des Dialysezentrums ist in den meisten Fällen untersagt, da ein nicht zertifiziertes Gerät im Falle eines Defekts, einen Stromausfall innerhalb des Dialysezentrums bewirken könnte bzw. eine Sicherung im Dialysezentrum zerstören könnte, mit entsprechenden negativen oder sogar gefährlichen Auswirkungen auf die zu diesem Zeitpunkt therapierten Patienten. Darüber hinaus könnten durch ein solches Gerät ausgelöste Kurzschlüsse auch zu einem Brand führen.

Daher ist ein Patient auf die Akkukapazität seines Gerätes angewiesen, um sich die Wartezeit während der Dialyse durch die Nutzung seines eigenen mobilen Endgerätes angenehmer zu gestalten. Erreicht die Akkukapazität ihr Minimum und hat der Patient keine Auflademöglichkeit dabei, ist es nicht möglich, sein Gerät weiterhin zu nutzen. Dadurch verschlechtert sich der Gemütszustand des Patienten mitunter drastisch, da er nun keine gewünschte Freizeitaktivität ausführen kann. Außerdem kann er dann nicht im Falle eines entleerten Smartphone-Akkus Kontakt mit anderen Personen aufnehmen oder diese mit ihm. Diese Personen könnten beispielsweise Verwandte oder Freunde sein, welche den Patienten nach der Therapie abholen müssen und sich gegebenenfalls verspäten.

Ein Dialysezentrum geht weiterhin ein Risiko ein, sollte es Patienten erlauben, ihr eigenes Ladegerät an das hauseigene Stromnetz anzuschließen. Insbesondere qualitativ minderwertige oder defekte Produkte können im schlimmsten Fall zu einem Stromausfall innerhalb des Zentrums führen. Des Weiteren ist der Patient, sollte er sein eigenes Gerät nutzen können, darauf angewiesen, dass sich eine Steckdose in passender Entfernung zu seinem Dialyseplatz befindet. Zudem muss der Patient das Gerät durchgehend in seinen Händen halten, um beispielsweise eine Serie damit schauen zu können. Oft kann nicht genau gesagt werden, auf welcher Seite der Maschinen sich der Patient befindet. Dementsprechend könnte ein Kabel störend im Weg liegen oder zu kurz sein, um sich von der Steckdose bis zu dem Patienten zu erstrecken.

Die WO 2017/064 251 A1 beschreibt ein medizinisches System, welches es einem Patienten ermöglicht, ein externes elektronisches Endgerät während der Behandlung sicher mit Strom zu versorgen. Hierbei ist eine elektrisch isolierte Steckdose am Maschinengehäuse vorgesehen, sodass über ein Kabel mobile Endgeräte aufgeladen werden können.

Die DE 10 2012 020 945 A1 beschreibt, wie ein mobiles Endgerät an einem Fluidmanagementgerät angebracht werden kann, um eine Anzeige- und Eingabeeinheit zu ersetzen. Allerdings steht dem Patienten dann das mobile Endgerät nicht mehr zu Unterhaltungszwecken zur Verfügung.

Die WO 2017 131 796 A1 offenbart ein multifunktionelles Schwesternrufsystem, an welches ein mobiles Endgerät angeschlossen werden kann. Allerdings können damit keine medizinischen Geräte ausgelesen oder angesteuert werden. Des Weiteren ist hierbei keine drahtlose Energieübertragung zum Aufladen des mobilen Endgeräts vorgesehen.

Die CN 108 186 232 A beschreibt eine Patientenliege, die eine Ablagefläche aufweist, auf welcher mobile Endgeräte drahtlos aufgeladen werden können.

Die CN 107 948 370 A beschreibt eine Endgeräthalterung mit integriertem Akku zur Fixierung und Aufladung mobiler Endgeräte. Die US 2015/0234363 (A1) und US 2011/0275984 (A1) beschreiben weitere aus dem Stand der Technik bekannte Aufnahmevorrichtungen für elektronische Endgeräte.

### Kurzbeschreibung der Erfindung

Es ist daher die Aufgabe der vorliegenden Erfindung, die Nachteile aus dem Stand der Technik zu vermeiden oder wenigstens zu mildern. Insbesondere soll eine Halterungsvorrichtung bereitgestellt werden, welche dazu ausgebildet ist, ein elektronisches Endgerät während der Dialysebehandlung zu halten und dieses gleichzeitig und sicher mit Strom zu versorgen.

Unter der genannten medizinischen Behandlungseinrichtungen, vorzugsweise Dialysemaschine, werden unter anderem herkömmliche Hämodialyse- bzw. Hämodiafiltrationsgeräte verstanden. Des Weiteren kann die Halterungsvorrichtung auch in Kombination mit weiteren Geräten verwendet werden, wie beispielsweise Peritonealdialysegeräte, Akutdialysegeräte oder Apheresegeräte, usw. Nachfolgend ist der Einfachheit halber die medizinische Behandlungseinrichtung durch den Begriff Dialysemaschine ersetzt.

Diese Aufgabe wird erfindungsgemäß dadurch gelöst, dass eine Halterungsvorrichtung einer Dialysemaschine zum Halten und Fixieren zumindest eines elektronischen Endgeräts, insbesondere der Unterhaltungselektronikbauart während einer Dialysebehandlung, welche an einer Dialysemaschine angebracht oder anbringbar ist, mit einer integrierten Ladevorrichtung, die dazu vorgesehen und ausgebildet ist, das gehaltene und/oder fixierte elektronische Endgerät mit Strom aus der Dialysemaschine zu versorgen, wobei eine Schutzschaltung in der Haltevorrichtung integriert ist, um einen Defekt eines gerade angeschlossenen Endgeräts zu erkennen.

In anderen Worten ausgedrückt ist innerhalb der Halterungsvorrichtung die Sicherungsschaltung vorgesehen, die elektrische Sicherungsmechanismen aufweist, welche das mobile Endgerät und die Dialysemaschine vor Überspannung, Unterspannung, Kurzschlüssen oder thermischer Beschädigung, beispielsweise Temperatursensoren, bewahren. Hierbei dient die Schutzschaltung dazu, das eingeklemmte/verwendete mobile Endgerät zu überwachen, um sowohl das mobile Endgerät, den Patienten als auch die Dialysemaschine und deren Umgebung zu schützen. Das Anbringen der Schutzschaltung in der Halterungsvorrichtung hat den Vorteil, dass durch diese Entkopplung kein Schaden an der Dialysemaschine durch das mobile Endgerät entstehen kann.

Alternativ oder zusätzlich ist es bevorzugt, wenn die Halterungsvorrichtung bereits mit Niederspannung von beispielsweise 5V zum Laden des Endgeräts versorgt wird. Auf diese Weise könnte eine Schutzschaltung, die vorzugsweise nur den Ladestrom überwacht, in der Behandlungsvorrichtung integriert sein. Für den Fall, dass ein Akku verwendet wird, kämen dann zwei Schutzschaltungen zum Einsatz. Hierbei sind eine erste Schutzschaltung in der Haltevorrichtung zur Überwachung des Ladevorgangs des mobilen Endgeräts und eine zweite Schutzschaltung in der medizinischen Behandlungsvorrichtung zur Überwachung des Ladevorgangs des Akkus vorgesehen.

In anderen Worten, soll dem Patienten die Möglichkeit gegeben werden, durch eine mit der Maschine verbundene Halterungsvorrichtung sein mobiles Endgerät sicher mit Strom zu versorgen. Hierbei ist das Installieren einer mit der Maschine verbundenen Halterungsvorrichtung für mobile Endgeräte, wie beispielsweise Smartphones, Tablets, E-Reader, usw., vorgesehen. Die Halterungsvorrichtung ermöglicht neben der Stromversorgung auch das Laden der mitgebrachten mobilen Endgeräte während der Dialysebehandlung.

Diese Lösung bringt den Vorteil mit sich, dass Patienten ihr mobiles Endgerät jederzeit aufladen können und sich damit die Zeit während der Dialysebehandlung angenehmer gestaltet können. Die Patienten müssen sich nicht um einen Ausfall ihrer mobilen Endgeräte auf Grund fehlender Akkukapazität sorgen. Darüber hinaus muss sich das Dialysezentrum keine Gedanken über die Nutzung eigens mitgebrachter Ladegeräte am Hausnetz machen. In anderen Worten hat dies den Vorteil, eine externe Stromversorgung ohne Sicherheitseinbußen zur Verfügung zu stellen.

Die Halterungsvorrichtung ist vorzugsweise mit einer Frontklappe und einer Rückklappe ausgebildet, welche über ein Gelenk/Scharnier auf einer Seite miteinander verbunden und zueinander verschwenkbar sind. Die Frontklappe ist durch einen Rahmen mit geschlossener Rückseite ausgebildet. Der Rahmen ist derart ausgebildet, um das mobile Endgerät aufzunehmen. Auf der gegenüberliegenden Seite des Gelenks ist die Frontklappe mit der Rückklappe über einen (Längen-) verstellbaren Boden verbunden. Dies ermöglicht es dem Patienten, die Halterungsvorrichtung auf eine ebene Unterlage/Fläche mit einem gewünschten Winkel zu stellen.

Der verstellbare Boden ist vorzugsweise mittels einer gerippten/gerillten Oberfläche oder einer Stellschraube ausgebildet, um einen Aufstellwinkel des mobilen Endgeräts flexibel einzustellen. Die Stellschraube ist demnach derart in dem Boden integriert ausgebildet, dass diese flexibel verschoben und festgezogen wird. Das Verschieben der Stellschraube ist beispielsweise über eine in dem Boden integrierte Schiene vorgesehen. Darüber hinaus ist es vorgesehen, dass der Boden über eine entsprechende Länge/Tiefe verfügt, um auch einen großen Aufstellwinkel, vorzugsweise größer als 90°, weiter bevorzugt größer als 120°, zu nutzen. Dies hat den Vorteil, dass die Halterungsvorrichtung auch auf einer weichen Unterlage wie beispielsweise einer Matratze stabil steht.

Vorteilhafte Ausgestaltungen sind in den Unteransprüchen beansprucht und werden nachstehend näher erläutert.

Es ist bevorzugt, wenn die Halterungsvorrichtung, das elektronische Endgerät und die Dialysemaschine dazu ausgebildet und vorgesehen sind, miteinander zu kommunizieren, um Daten zwischen der Dialysemaschine und dem elektronischen Endgerät, vorzugsweise bidirektional, zu übertragen. In anderen Worten ausgedrückt, ist die Halterungsvorrichtung als eine intelligente Halterungsvorrichtung ausgebildet, welche einen Austausch von Daten bzw. eine Kommunikationsverbindung zwischen der Dialysemaschine und dem mobilen Endgerät vorsieht.

Es ist bevorzugt, wenn die zu übertragenden Daten behandlungsspezifische Daten/Informationen sind. Die Eingabe- und Anzeigevorrichtung weist unterschiedliche Anzeigeelemente und Schaltflächen auf, um Daten/Informationen einzugeben und anzuzeigen. Dementsprechend kann ein Anzeigeelement bspw. den Fortschritt der Dialysebehandlung darstellen und mittels einer Schaltfläche das medizinische Personal gerufen werden.

Des Weiteren sind unter den zu übertragenden Daten, bspw. Informationen vom Patienten zu dessen Flüssigkeitsaufnahme während der Dialysebehandlung zu verstehen. Diese können wiederum über Schaltflächen der Eingabe- und Anzeigevorrichtung eingegeben werden. Da es ein Ziel der Dialysebehandlung ist, dem Patienten überschüssiges Wasser zu entziehen, kann die Flüssigkeitsaufnahme direkt berücksichtigt werden, indem das Ultrafiltrationsvolumen um das Volumen der aufgenommenen Flüssigkeit erhöht wird. Ein weiterer Vorteil ist, dass das zusätzlich benötigte Ultrafiltrationsvolumen gleichmäßig auf die verbleibende Therapiedauer aufgeteilt werden kann.

Diese integrierbare Komfortfunktion wie Auslesen des Therapieverlaufs und die direkte und sofortige Informationsübertragung zwischen der Dialysemaschine und dem mobilen Endgerät bzw. der Halterungsvorrichtung bieten eine effiziente und für den Patienten angenehmere Dialysebehandlung.

So kann beispielsweise eine Schaltfläche für ein Getränk mit einem vorbestimmten Volumen von bis zu 150ml, eine weitere Schaltfläche für ein mittelgroßes Getränk mit einem Volumen zwischen 150ml und 250ml, eine weitere Schaltfläche für ein großes Getränk mit einem Volumen von mehr als 250ml (bis 400ml) auf der Anzeigevorrichtung zur Verfügung stehen. Denkbar wäre natürlich auch eine direkte Erfassung des Volumens der Getränke durch eine numerische Eingabe oder durch die Verwendung einer als Schieberegler ausgebildeten Schaltfläche.

Darüber hinaus ist es bevorzugt, wenn mit der Einnahme von Speisen während der Behandlung auf ähnliche Weise verfahren wird, um direkt Anpassungen vorzunehmen. Insbesondere, wenn der Patient beispielsweise Obst- oder Gebäckstücke oder Ähnliches zu sich nimmt/isst, von dem zumindest näherungsweise bekannt ist, wie viel Kalium und/oder Natrium darin enthalten ist, kann der Patient dies über die Anzeige- und Eingabevorrichtung an die Dialysemaschine weitergeben. Diese ist daraufhin dazu ausgebildet, die Zusammensetzung der Dialysierflüssigkeit so anzupassen, dass die durch die Nahrung zusätzlich aufgenommene Menge an Kalium und/oder Natrium und/oder sonstigem Elektrolyt, welches in frischer Dialysierflüssigkeit enthalten ist, diffusiv im weiteren Verlauf der Behandlung wieder entzogen wird.

Es ist weiter bevorzugt, dass das Endgerät auch für die Patientenidentifikation eingesetzt werden kann und damit z.B. die Funktion einer aus dem Stand der Technik bekannten Patientenkarte mit übernimmt. Auf Basis dieser Identifikation kann dann beispielsweise der Download von Behandlungsparametern aus dem Datenmanagementsystem des Dialysezentrums an die Dialysemaschine erfolgen. Alternativ könnten diese Parameter/Daten auch direkt auf dem Endgerät gespeichert und von dort an die Dialysemaschine übermittelt werden.

Es ist bevorzugt, wenn die Halterungsvorrichtung dazu vorgesehen und ausgebildet ist, ein mobiles Endgerät einzuspannen, um vorzugsweise mittels eines Schwenkarms/Haltearms in verschiedene Positionshaltungen gebracht zu werden und in der eingestellten Positionshaltung zu verbleiben. Mit anderen Worten heißt das, dass die Halterungsvorrichtung vorzugsweise zwei Spannelemente aufweist, welche an unterschiedlich große Endgeräte anpassbar sind und derart ausgebildet sind, dass das mobile Endgerät aufgrund des durch die Spannelemente auf das Endgerät ausgeübten Drucks, von der Halterungsvorrichtung gehalten und fixiert werden kann. Die Spannelemente können mittels Positionshalter, welche am Rand aus der Halterungsvorrichtung herausragen, von dem Patienten händisch verschoben werden, um unterschiedlich große Endgeräte einzuspannen. Dies hat den Vorteil, dass die Art des Einspannens für das Endgerät schonend ist und keinen Schaden hinterlässt.

Vorteilhaft ist es, wenn die Halterungsvorrichtung an einem mehrgelenkigen und schwenkbaren Schwenkarm/Haltearm angebracht ist. Der Schwenkarm ist hierbei als Teil der Dialysemaschine ausgebildet. Der Schwenkarm ermöglicht es dem Patienten, das in die Halterungsvorrichtung eingespannte mobile Endgerät derart vor sich zu platzieren, dass es weder gehalten noch abgelegt werden muss.

Des Weiteren hat dies den Vorteil, dass der Patient das mobile Endgerät nicht durchgehend in den Händen halten muss, da die Form der Halterungsvorrichtung so konzipiert ist, dass diese selbstständig stehen und/oder beispielsweise auf dem Schoß platziert werden kann. Durch diese ergonomische und komfortable Halterungsvorrichtung können Patienten das Gerät für sie gemütlich platzieren, ohne es durchgehend halten zu müssen.

Es ist bevorzugt, wenn ein Bildschirm des elektronischen Endgeräts als die Eingabe-/ Anzeigevorrichtung vorgesehen und ausgebildet ist und während einer Behandlung frei zugänglich für den Anwender und/oder Patienten ist. Dem Patienten wird die Möglichkeit gegeben, durch diese mit der Maschine verbundene Halterungsvorrichtung für das mobile Endgerät, dieses sicher mit Strom zu versorgen und das mobile Endgerät trotzdem in einer für ihn gemütlichen und gut zugänglichen Lage zu fixieren, um es als Unterhaltungsmittel weiter nutzen zu können.

Die Halterungsvorrichtung ist dabei so konzipiert, dass für den Patienten weiterhin eine volle Bedienung des mobilen Endgeräts gewährleistet ist. Dies hat den Vorteil, dass sein allgemeines Empfinden und die Qualität der verbrachten Dialysezeit steigt, sofern er seine Zeit mit einem mobilen Endgerät verbringen möchte.

Es ist bevorzugt, wenn die Halterungsvorrichtung dazu vorgesehen und ausgebildet ist, das mobile Endgerät über ein Kabel, welches mit der Dialysemaschine verbunden ist, oder über eine Energiespeichereinheit vorzugweise einen Akku, mit Strom zu versorgen. In dem Fall, dass die Halterungsvorrichtung über eine separate Energiespeichereinheit verfügt, wird die Energiespeichereinheit vorzugsweise zwischen zwei Behandlungen geladen.

Ferner ist es bevorzugt, wenn die Ladehalterung einen drahtlosen Lademechanismus/eine Energieübertragungsvorrichtung aufweist. Die Ladehalterung ist vorzugsweise auf einer Seite der Dialysemaschine angebracht. In anderen Worten kann für das Aufladen der Energiespeichereinheit beispielsweise ein kabelloses Verfahren, vorzugsweise mittels Induktion, angewendet werden. Der Lademechanismus kann auch in Form einer weiteren Halterung/Ladehalterung an der Dialysemaschine angebracht sein. Auf diese Weise kann die Energiespeichereinheit der Halterungsvorrichtung zwischen jeder Therapie an die Ladehalterung der Dialysemaschine angedockt werden, um so für die nächste Therapie wieder aufgeladen zu sein.

Die Ladehalterung ermöglicht eine kraft- und/oder formschlüssige Befestigung der Halterungsvorrichtung an der Dialysemaschine. Dazu zählen beispielsweise Steckverbindungen, Klemmen oder auch Magnetverbindungen, eine Tasche oder Klemmen/Haken, in welche die Halterungsvorrichtung zum Laden der darin integrierten Energiespeichereinheit hineingelegt/angebracht/befestigt wird.

Ferner ist es bevorzugt, wenn der Lademechanismus sich innerhalb oder außerhalb des Gehäuses der Dialysemaschine befindet und mindestens eine Spule darin integriert ist, die für die drahtlose Energieübertragung mittels induktiver bzw. resonanter induktiver Kopplung verwendet wird/nötig ist. Dabei sind gängige Maßnahmen zur Abschirmung vorgesehen bzw. implementiert, sodass eine elektromagnetische Verträglichkeit gewährleistet ist und umliegende medizinische sowie nicht medizinische Geräte weder stören noch gestört werden.

Weiter bevorzugt ist eine Ladehalterung, die an einer Dialysemaschine angebracht ist. Die Ladehalterung zur drahtlosen Energieübertragung hat eine integrierte Spule, die sich am Gehäuse der Dialysemaschine über ein Scharnier ausklappbar befindet. Im ausgeklappten Zustand kann ein mittels Induktion ladefähiges mobiles Endgerät (keine zusätzliche Halterungsvorrichtung nötig) oder die Halterungsvorrichtung zum Laden auf die Ladehalterung gelegt werden. Sobald kein Laden mehr nötig ist, kann die Ladehalterung eingeklappt werden.

Es ist bevorzugt, wenn die Halterungsvorrichtung zumindest eine integrierte Schaltung aufweist. Die Schaltung umfasst eine Ladeschaltung und eine Verarbeitungsschaltung und/oder eine Sicherungsschaltung.

In dem beanspruchten Fall, dass die Halterungsvorrichtung an der Dialysemaschine angebracht ist, ist innerhalb der Halterungsvorrichtung die Sicherungsschaltung vorgesehen, die elektrische Sicherungsmechanismen aufweist, welche das mobile Endgerät und die Dialysemaschine vor Überspannung, Unterspannung, Kurzschlüssen oder thermischer Beschädigung bewahren.

In dem nicht beanspruchten Fall, dass die Halterungsvorrichtung an einer Patientenliege angebracht ist, ist keine Absicherung zwischen der Dialysemaschine und der Halterungsvorrichtung notwendig, da die Stromversorgung der Halterungsvorrichtung mit der Patientenliege vorgesehen ist.

In dem Fall, dass die Halterungsvorrichtung über eine Energiespeichereinheit gespeist wird, befinden sich innerhalb der Halterungsvorrichtung elektrische Sicherungsmechanismen, welche das mobile Endgerät vor Überspannung, Unterspannung, Kurzschlüssen und/oder thermischer Beschädigung bewahren. Weiterhin befinden sich innerhalb der Halterungsvorrichtung alle benötigten Schaltungen für die korrekte Ansteuerung des verbauten Akkus und dessen Ladeschaltung. In diesem Fall hat die Erfindung den Vorteil, dass sich an der Halterungsvorrichtung kein störendes Kabel befindet und eine Ladehalterung für ein schnelles Laden ohne Kabel sorgt.

Somit übernimmt die Halterungsvorrichtung Sicherheitsüberwachungen und schützt mobile Endgeräte sowie die Dialysemaschine vor elektrischen oder thermischen Gefahren.

In einer bevorzugten Ausführungsform der Erfindung ist die Ladevorrichtung mit integrierten Adaptern/Ladeadaptern, beispielsweise USB-Verbindungen, ausgebildet, um verschiedene Modelle von elektronischen Endgeräten mit der Ladevorrichtung elektrisch verbinden zu können. Mit anderen Worten bedeutet das, dass die Halterungsvorrichtung das Laden der mitgebrachten mobilen Endgeräte durch integrierte Ladeadapter für verschiedene Standards der mobilen Endgeräte ermöglicht.

Es ist bevorzugt, wenn die Halterungsvorrichtung einen Kopfhöreranschluss aufweist. In anderen Worten bedeutet das, dass an der Halterungsvorrichtung ein externer Anschluss/eine externe Verbindung zum Einstecken von Kopfhörer vorgesehen ist.

Es ist bevorzugt, wenn die Halterungsvorrichtung dazu vorgesehen und ausgebildet ist, um über NFC (Near Field Communication, ein auf RFID-Technik basierender internationaler Übertragungsstandard zum kontaktlosen Austausch von Daten) und eine entsprechende App mit dem angeschlossenen elektronischen Endgerät zu kommunizieren und Daten auszutauschen. In anderen Worten kann die Halterungsvorrichtung über NFC und eine entsprechende App mit dem angeschlossenen mobilen Endgerät kommunizieren und Informationen über den Therapieverlauf austauschen. Die vom Patienten gegebenenfalls eingegebene Information, ob dieser beispielsweise Flüssigkeit während der Behandlung zu sich genommen hat, kann direkt an die Maschine für eine Anpassung der Therapie bzw. von Therapieparametern, insbesondere des Ultrafiltrationsvolumens, übermittelt werden. Die Übermittlung erfolgt vorzugsweise per Funk oder Bluetooth. Auf diese Weise kann ebenfalls eine Funktion für den Personalruf mit in der Halterungsvorrichtung integriert werden. Hierbei ist es bevorzugt, basierend auf einer Eingabe am elektronischen Endgerät über die aufgenommene Flüssigkeitsmenge des Patienten das Ultrafiltrationsvolumen direkt, vorzugsweise automatisch, anzupassen.

Die vorliegende Erfindung betrifft ferner eine Ladevorrichtung mit einer integralen/integrierten Stromversorgungseinheit an einer Dialysemaschine mit einer Halterungevorrichtung gemäß einem der vorhergehenden Aspekte. In anderen Worten ausgedrückt, dient die Ladevorrichtung zugleich als eine Stromversorgungseinheit an einer Dialysemaschine mit einer Halterungsvorrichtung.

Zusammenfassend sieht die vorliegende Erfindung eine Halterungsvorrichtung/Endgeräthalterung vor, in welche sich möglichst viele der gängigen Modelle von mobilen Geräten fixieren lassen. Dabei bleibt der Bildschirm frei, um die Bedienung der mobilen Endgeräte weiterhin zu ermöglichen. Des Weiteren ist es vorgesehen, dass die fixierten Endgeräte über integrierte Adapter innerhalb der Halterungsvorrichtung für verschiedene Standards mit Strom versorgt werden. Weiterhin befinden sich Schaltungen für die elektrische und thermische Sicherheit (Temperatursensoren) im Inneren der Halterungsvorrichtung. Die Halterungsvorrichtung kann über ein Kabel mit der Maschine verbunden und mit Strom versorgt werden. Alternativ kann die Stromversorgung auch via Akku und einer kabellosen Ladehalterung, welche vorzugsweise an der Maschine oder an der Patientenliege befestigt ist, realisiert sein.

Dabei wird das Design so gewählt, dass die Halterungsvorrichtung eigenständig stehen und in verschiedene Positionen gesetzt werden kann, um so die Notwendigkeit des durchgehenden Festhaltens durch den/die Patienten zu beseitigen. Alternativ wird die Halterungsvorrichtung an einem Schwenkarm angebracht, welcher Teil der Maschine ist. Anschlüsse für Kopfhörer können ebenso wie Schnelleingabe-Tasten zusätzlich integriert werden. Über die Schnelleingabe-Tasten kann beispielsweise die Lautstärke eingestellt werden oder eine Vor-/Zurücktaste betätigt werden.

Erweitern lässt sich das System durch eine kabellose Schnittstelle, über welche Halterungsvorrichtung, Dialysemaschine und mobiles Endgerät (NFC, Bluetooth, WLAN, oder Ähnliche) miteinander kommunizieren und Informationen über den Therapieverlauf austauschen können.

### Kurzbeschreibung der Figuren

Fig. 1 ist eine schematische Darstellung einer Halterungsvorrichtung in Kombination mit einem Smartphone;
Fig. 2 ist eine schematische Darstellung einer Halterungsvorrichtung in Kombination mit einem Tablet;
Fig. 3 ist eine schematische Seitenansicht der Halterungsvorrichtung;
Fig. 4 ist eine schematische Darstellung einer ersten Ladehalterung angebracht an einer Dialysemaschine;
Fig. 5 ist eine schematische Darstellung einer zweiten Ladehalterung angebracht an einer Dialysemaschine;
Fig. 6 ist eine schematische Darstellung der ersten Ladehalterung angebracht an einer Dialysemaschine mit Halterungsvorrichtung;
Fig. 7 ist eine schematische Darstellung der zweiten Ladehalterung angebracht an einer Dialysemaschine mit Halterungsvorrichtung;
Fig. 8 ist eine schematische Darstellung einer dritten Ladehalterung angebracht an einer Dialysemaschine;
Fig. 9 ist eine schematische Darstellung einer Halterungsvorrichtung mit einem Schwenkarm an einer Dialysemaschine; und
Fig. 10 ist eine schematische Darstellung der Benutzeroberfläche der Anzeige- und Eingabevorrichtung.

### Beschreibung der Ausführungsformen

Die nachfolgend erwähnten Ausführungsformen in Zusammenhang mit einer Patientenliege fallen nicht unter den beanspruchten Gegenstand der Ansprüche.

Nachstehend werden Ausführungsformen der vorliegenden Offenbarung auf der Basis der zugehörigen Figuren beschrieben. Die Figuren sind lediglich von schematischer Natur und dienen dem Verständnis der Erfindung. Die gleichen Elemente sind mit denselben Bezugszeichen bezeichnet.

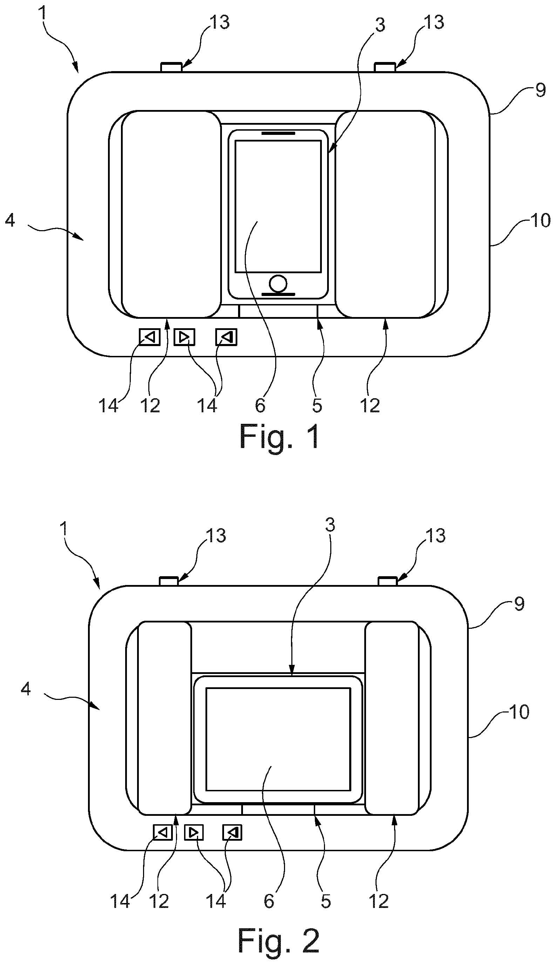

Fig. 1 zeigt eine schematische Darstellung einer Halterungsvorrichtung 1 in Kombination mit einem Smartphone als mobiles Endgerät 3 und Fig. 2 zeigt eine schematische Darstellung der Halterungsvorrichtung 1 in Kombination mit einem Tablet als mobiles Endgerät 3. In Fig. 1 und Fig. 2 ist die Halterungsvorrichtung 1 für eine Dialysemaschine 2 dazu vorgesehen, das mobile Endgerät 3 zu halten und zu fixieren.

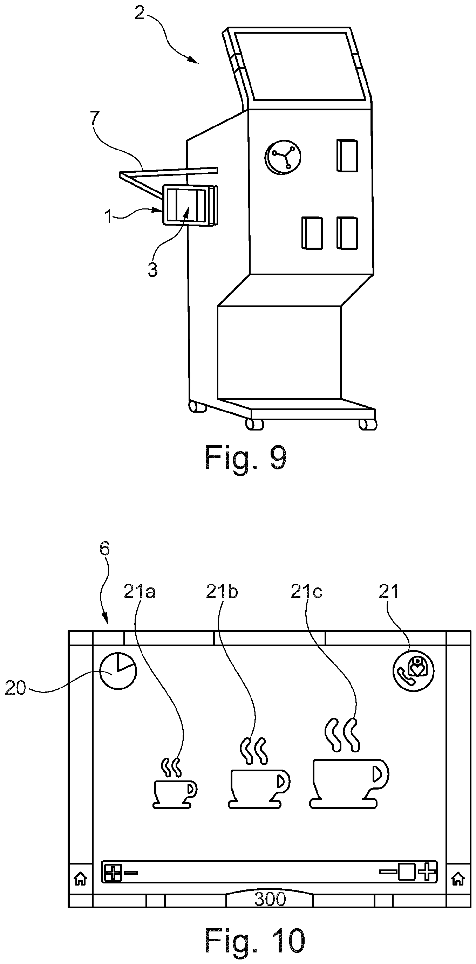

Die Halterungsvorrichtung 1 kann an der Dialysemaschine 2 gemäß einer ersten Ausführungsform (siehe Fig. 9) oder an einer Patientenliege gemäß einer zweiten Ausführungsform (nicht beansprucht) angebracht sein. Gemäß der zweiten Ausführungsform ist die Anbringung der Halterungsvorrichtung 1 vorzugsweise an der Armlehne/-stütze vorgesehen.

Die Halterungsvorrichtung 1, wie in Fig. 1 und Fig. 2 gezeigt, hat einen Rahmen 4 mit einer geschlossenen Rückseite (nachstehend auch als Vorderklappe bezeichnet). Der Rahmen 4 nimmt das mobile Endgerät 3, Spannelemente 12 und eine integrierte Ladevorrichtung 5 auf, welche zumindest einen Ladeadapter (nicht dargestellt) aufweist, um das mobile Endgerät 3 mit der Ladevorrichtung 5 zu verbinden. Die Anzeige- und Eingabevorrichtung 6 wird durch das/den Display/Bildschirm des mobilen Endgeräts 3 bereitgestellt. Das mobile Endgerät 3 ist mittels zumindest eines Spannelements 12, vorzugsweise zwei, in der Halterungsvorrichtung 1 eingespannt. Die beiden Spannelemente 12 sind vorzugsweise seitlich von dem mobilen Endgerät 3 angeordnet, und sind durch seitliches Verschieben dazu ausgebildet, sich an die Größe des einzuspannenden mobilen Endgeräts 3 anzupassen.

Alternativ ist auch das Verwenden von nur einem Spannelement 12 denkbar. Als weitere Alternative ist das Verwenden von zumindest einem Spannelement 12 denkbar, welches oberhalb und/oder unterhalb des mobilen Endgeräts 3 angeordnet ist und sich die Ladevorrichtung 5 dementsprechend auf einer Seite der Halterungsvorrichtung 1 befindet. Darüber hinaus kann zusätzlich oder alternativ auch eine magnetische Halterung des mobilen Endgerätes vorgesehen sein.

Die Halterungsvorrichtung 1 ist mit einem Anschluss 9, vorzugsweise für ein Versorgungskabel, ausgebildet. Der Anschluss 9 verbindet die Halterungsvorrichtung 1 mit der Dialysemaschine 2 gemäß der ersten Ausführungsform. In der zweiten Ausführungsform verbindet das Kabel die Halterungsvorrichtung 1 mit der Patientenliege. Der Anschluss 9 sorgt in beiden Ausführungsformen für die Stromzufuhr zu der Halterungsvorrichtung 1, um das gehaltene und/oder fixierte elektronische Endgerät 3 mit Strom zu versorgen.

Die Halterungsvorrichtung 1 hat an der oberen Kante des Rahmens 4 der Halterungsvorrichtung 1 zumindest einen Positionshalter 13. Wie in Fig. 1 gezeigt, weist die Halterungsvorrichtung 1 zwei Positionshalter 13 auf. Mit diesen Positionshaltern 13 ist es dem Patienten möglich, durch Verschieben von Spannelementen 12 diese so einzustellen, dass das mobile Endgerät gehalten und fixiert ist.

Der Rahmen 4 der Halterungsvorrichtung 1 weist zumindest eine Schnelleingabe-Taste 14 auf. Die Schnelleingabe-Tasten 14 gemäß den Fign. 1 und 2 sind vorzugsweise am unteren Rahmen 4 angebracht. Die Schnelleingabe-Tasten 14 können beispielsweise die Lautstärke und/oder eine Vor- /Zurücktaste betreffen. Alternativ ist auch denkbar, dass eine Schnelleingabe-Taste 14 als Schwesternruf fungiert.

In den Fign. 1 und 2 kann die Ladevorrichtung 5 alternativ als eine Speichereinheit 11 gemäß einer dritten Ausführungsform ausgebildet sein. Im Falle der dritten Ausführungsform entfällt der Anschluss 9 sowie das zugehörige Versorgungskabel wie später beschrieben.

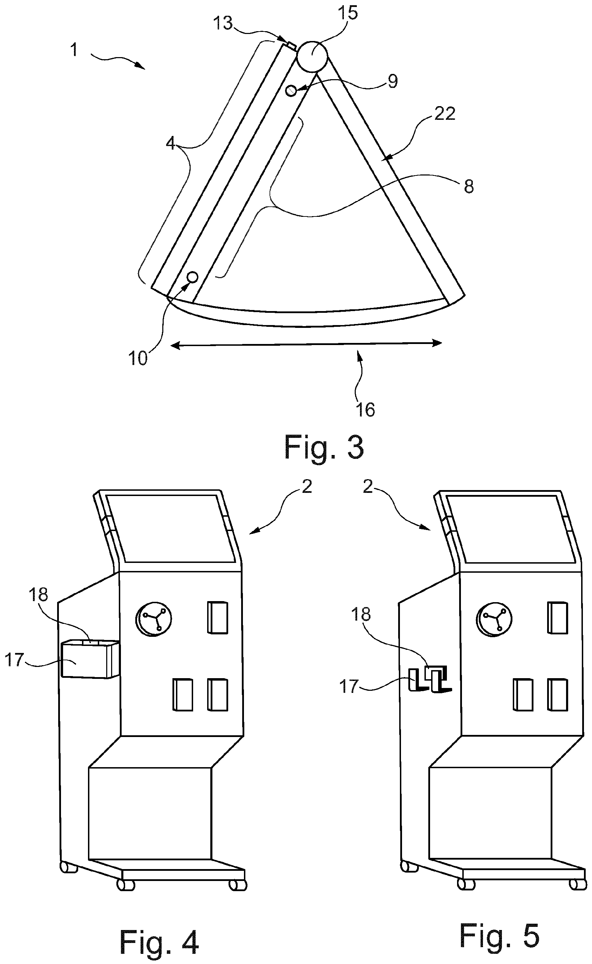

Fig. 3 ist eine schematische Seitenansicht der Halterungsvorrichtung 1. In Fig. 3 ist der Rahmen 4 der Halterungsvorrichtung 1 gezeigt. Die Halterungsvorrichtung 1 hat eine durch den Rahmen 4 gebildete Vorderklappe 4 und eine Rückklappe 22. Die Vorderklappe 4 ist über ein Gelenk 15 mit der dazu verschwenkbaren/wegklappbaren Rückklappe 22 ausgebildet und in der Vorderklappe 4 ist die Schaltung 8 untergebracht/integriert. Die Vorderklappe 4 und die Rückklappe 22 sind auf der gegenüberliegenden Seite des Gelenks 15 über einen (längen-) verstellbaren Boden 16 verbunden. Somit ist ein Abstellen der Halterungsvorrichtung 1 gemäß der dritten Ausführungsform auf einer ebenen Unterlage möglich.

Die Vorderklappe 4 aus Fig. 3 zeigt den Kopfhöreranschluss 10 und den Anschluss 9 für das (Versorgungs-) Kabel. In der Vorderklappe 4 ist zugleich eine Schaltung 8 vorgesehen. Die Schaltung 8 weist eine Ladeschaltung und Verarbeitungsschaltung und/oder Sicherungsschaltung auf.

Die nachstehenden Figuren 4 bis 7 zeigen Beispiele für Maschinen zur Blutbehandlung/Dialysemaschinen 2 mit Ladehalterungen 17 für Halterungsvorrichtungen 1 gemäß der dritten Ausführungsform. Dabei weisen die Ladehalterungen 17 drahtlose Lademechanismen/Energieübertragungsvorrichtungen 18 auf. Die Figuren 4 bis 8 zeigen auf der linken Seite der Dialysemaschine 2 jeweils eine Ladehalterung 17.

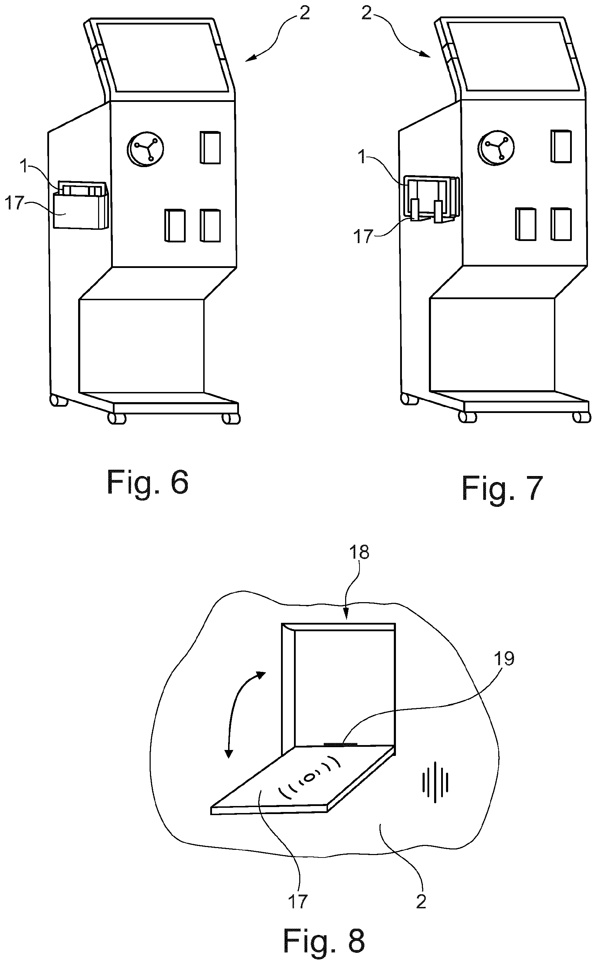

Für die Ladehalterung 17 sind verschiedene Ausführungsformen denkbar, die eine kraft- und/oder formschlüssige Befestigung der Halterungsvorrichtung 1 an der Dialysemaschine 2 ermöglichen. Dazu zählen beispielsweise Steckverbindungen, Klemmen oder auch Magnetverbindungen. In Fig. 4 ist eine Ladehalterung 17 gezeigt, welche als eine Tasche ausgebildet ist, in welche die Halterungsvorrichtung 1 zum Laden der darin integrierten Energiespeichereinheit 11 hineingelegt wird. In Fig. 6 ist die taschenförmige Ladehalterung 17 mit der sich darin befindlichen Halterungsvorrichtung 1 gezeigt.

In Fig. 5 ist eine Ladehalterung 17 gezeigt, welche als zwei Klemmen/Haken ausgebildet ist, in welche die Halterungsvorrichtung 1 zum Laden der darin integrierten Energiespeichereinheit 11 hineingelegt wird. In Fig. 7 ist die klemmen-/hakenförmige Ladehalterung 17 mit der sich darin befindlichen Halterungsvorrichtung 1 gezeigt.

Der Lademechanismus 18 der Fig. 4 bis 7 können sich sowohl innerhalb als auch außerhalb des Gehäuses der Dialysemaschine 2 befinden und haben mindestens eine Spule integriert, die für die drahtlose Energieübertragung mittels induktiver bzw. resonanter induktiver Kopplung verwendet wird. Dabei sind gängige Maßnahmen zur Abschirmung vorgesehen bzw. implementiert, sodass eine elektromagnetische Verträglichkeit gewährleistet ist und umliegende medizinische sowie nicht medizinische Geräte weder stören noch gestört werden.

Die vorstehend beschriebenen Ladehalterungen 17 sind in ähnlicher oder gleicher Ausführung auch an einer Patientenliege, vorzugsweise an den Armstützen denkbar (nicht dargestellt).

Fig. 8 ist eine schematische Darstellung einer dritten Ladehalterung 17, angebracht an einer Dialysemaschine 2. Fig. 8 zeigt als zusätzliche Ausführungsform in vergrößerter Darstellung eine Ladehalterung 17 zur drahtlosen Energieübertragung mit integrierter Spule, die sich am Gehäuse der Dialysemaschine 2 oder an einer Patientenliege über ein Scharnier 19 ausklappbar befindet. Im ausgeklappten Zustand kann ein mittels Induktion ladefähiges mobiles Endgerät 3 zum Laden auf die Ladehalterung 17 gelegt werden. Hierbei ist keine zusätzliche Halterungsvorrichtung 1 nötig. Sobald kein Laden mehr nötig ist, kann die Ladehalterung 17 eingeklappt werden.

Alternativ kann die ausklappbare Ladehalterung 17 gemäß Fig. 8 dazu ausgebildet sein, die Halterungsvorrichtung 1 zu laden, sobald diese sich auf der ausgeklappten Ladehalterung 17 liegt.

Fig. 9 ist eine schematische Darstellung einer Halterungsvorrichtung 1 mit einem Schwenkarm 7 an der Dialysemaschine 2. Die Halterungsvorrichtung 1 ist an einem mehrgelenkigen und schwenkbaren Schwenkarm/Haltearm 7 angebracht. Der Schwenkarm 7 ist entweder als Teil der Dialysemaschine 2 gemäß der ersten Ausführungsform oder als Teil der Patientenliege gemäß der zweiten Ausführungsform (nicht dargestellt) ausgebildet. Der Schwenkarm 7 ermöglicht es dem Patienten, das in die Halterungsvorrichtung 1 eingespannte mobile Endgerät 3 derart vor sich zu platzieren, dass es weder abgelegt noch von ihm gehalten werden muss.

Fig. 10 ist eine schematische Darstellung der Benutzeroberfläche der Anzeige- und Eingabevorrichtung 6. In Fig. 10 ist beispielhaft eine mögliche Benutzeroberfläche einer App dargestellt, die auf dem mobilen Endgerät 3 ausgeführt wird. Die Benutzeroberfläche weist unterschiedliche Anzeigeelemente 20 und Schaltflächen 21 auf. Das Anzeigeelement 20 zeigt beispielsweise den Fortschritt der Dialysebehandlung an. Über die Schaltfläche 21 kann das medizinische Personal gerufen werden.

Des Weiteren besteht die Möglichkeit, mit den Schaltflächen 21a, b, c eine Flüssigkeitsaufnahme während einer Dialysebehandlung der Dialysemaschine 2 zu melden. In Fig. 10 sind die Schaltfläche 21a, b, c als unterschiedlich große Tassen Kaffee dargestellt, die jeweils für eine bestimmte Flüssigkeitsmenge stehen. Auf diese Weise kann der Patient die zu sich genommene Flüssigkeitsmenge durch Antippen der entsprechend großen Tasse der Dialysemaschine mitteilen.

Eine ähnliche Darstellung ist für beispielsweise für Nahrung denkbar. Dabei ist es bevorzugt, wenn der Patient zudem eingeben kann, welche Nahrung er zu sich genommen hat, sodass die Dialysebehandlung an die entsprechend zugenommenen Elektrolyte angepasst werden kann.

### Bezugszeichen

- 1.: Halterungsvorrichtung
- 2.: Dialysemaschine
- 3.: Endgerät
- 4.: Rahmen/Vorderklappe
- 5.: Ladevorrichtung
- 6.: Eingabe- und Anzeigevorrichtung
- 7.: Schwenkarm
- 8.: Schaltung
- 9.: Anschluss
- 10.: Kopfhöreranschluss
- 11.: Energiespeichereinheit
- 12.: Spannelemente
- 13.: Positionshalter
- 14.: Schnelleingabe-Tasten
- 15.: Gelenk
- 16.: verstellbarer Boden
- 17.: Ladehalterung
- 18.: Lademechanismus
- 19.: Scharnier
- 20.: Anzeigeelemente
- 21.: Schaltflächen
- 22.: Rückklappe

## Patentansprüche

1. Halterungsvorrichtung (1) einer Dialysemaschine (2) zum Halten und Fixieren zumindest eines elektronischen Endgeräts (3), insbesondere der Unterhaltungselektronikbauart während einer Dialysebehandlung, wobei die Halterungsvorrichtung (1) an der Dialysemaschine (2) angebracht oder anbringbar ist und wobei in der Halterungsvorrichtung eine Ladevorrichtung (5) integriert ist, die dazu vorgesehen und ausgebildet ist, das an der Dialysemaschine gehaltene und/oder fixierte elektronische Endgerät (3) mit Strom aus der Dialysemaschine (2) zu versorgen, **gekennzeichnet durch**
eine Schutzschaltung (8), die in der Haltevorrichtung (1) integriert ist, um einen Defekt eines gerade angeschlossenen Endgeräts (3) zu erkennen.

2. Halterungsvorrichtung (1) gemäß Anspruch 1, **dadurch gekennzeichnet, dass** das elektronische Endgerät (3) und die Dialysemaschine (2) dazu ausgebildet und vorgesehen sind, miteinander zu kommunizieren, um Daten zwischen der Dialysemaschine (2) und dem elektronischen Endgerät (3) mittels einer Eingabe- und Anzeigevorrichtung (6) des elektronischen Endgeräts (3), vorzugsweise bidirektional, zu übertragen.

3. Halterungsvorrichtung (1) gemäß einem vorhergehenden Anspruch, **dadurch gekennzeichnet, dass** die Halterungsvorrichtung (1) dazu vorgesehen und ausgebildet ist, ein mobiles Endgerät (3) einzuspannen, um vorzugsweise mittels eines Schwenkarms (7), in verschiedene Positionshaltungen gebracht zu werden und in der eingestellten Positionshaltung zu verbleiben.

4. Halterungsvorrichtung (1) gemäß Anspruch 2, **dadurch gekennzeichnet, dass** die Halterungsvorrichtung (1) derart ausgebildet ist, dass ein Bildschirm des elektronischen Endgeräts (3), der als die Eingabe-/ Anzeigevorrichtung (6) vorgesehen und ausgebildet ist, während einer Behandlung frei zugänglich für den Anwender und/oder Patienten ist.

5. Halterungsvorrichtung (1) gemäß einem vorhergehenden Anspruch, **dadurch gekennzeichnet, dass** die Halterungsvorrichtung (1) zumindest eine in die Halterungsvorrichtung integrierte Schaltung (8) aufweist, welche eine Verarbeitungs- und Ladeschaltung hat.

6. Halterungsvorrichtung (1) gemäß einem vorhergehenden Anspruch, **dadurch gekennzeichnet, dass** die Ladevorrichtung (5) mit integrierten Adaptern/Ladeadaptern, vorzugsweise USB-Verbindungen, ausgebildet ist, um verschiedene Modelle von elektronischen Endgeräten (3) mit der Ladevorrichtung (5) elektrisch zu verbinden.

7. Halterungsvorrichtung (1) gemäß einem vorhergehenden Anspruch, **dadurch gekennzeichnet, dass** die Halterungsvorrichtung (1) einen Kopfhöreranschluss (10) aufweist.

8. Halterungsvorrichtung (1) gemäß einem vorhergehenden Anspruch, **dadurch gekennzeichnet, dass** die Halterungsvorrichtung (1) dazu vorgesehen und ausgebildet ist, um über NFC und eine entsprechende App mit dem angeschlossenen elektronischen Endgerät (3) zu kommunizieren und Daten auszutauschen, vorzugsweise um basierend auf einer Eingabe am elektronischen Endgerät (3) über die aufgenommene Flüssigkeitsmenge des Patienten das Ultrafiltrationsvolumen direkt anzupassen.

9. Halterungsvorrichtung (1) gemäß einem vorhergehenden Anspruch, **dadurch gekennzeichnet, dass** die Halterungsvorrichtung (1) dazu vorgesehen und ausgebildet ist, das mobile Endgerät (3) über einen Anschluss (9) für ein Kabel, vorzugsweise ein Versorgungskabel, welches mit der Dialysemaschine (1) oder der Patientenliege (4) verbunden ist, oder über eine Energiespeichereinheit (11), vorzugweise einen Akku, mit Strom zu versorgen.

10. Ladevorrichtung (5) mit integraler Stromversorgungseinheit an einer Dialysemaschine (2) mit einer Halterungevorrichtung (1) gemäß einem der vorhergehenden Ansprüche.

## Claims

1. A holding device (1) of a dialysis machine (2) for holding and fixing at least one electronic terminal device (3), in particular of the entertainment electronics type, during a dialysis treatment, wherein the holding device (1) is or can be attached to the dialysis machine (2), and wherein a charging unit (5) is integrated in the holding device (1) which is provided and designed to supply the electronic terminal device (3) held and/or fixed to the dialysis machine with power from the dialysis machine (2), **characterized by**
a protection circuit (8) that is integrated in the holding device (1) to detect a defect of a terminal device (3) that has just been connected.

2. The holding device (1) according to claim 1, **characterized in that** the electronic terminal device (3) and the dialysis machine (2) are designed and provided to communicate with each other in order to transmit, preferably bidirectionally, data between the dialysis machine (2) and the electronic terminal device (3) by means of an input and display device (6) of the electronic terminal device (3).

3. The holding device (1) according to a preceding claim, **characterized in that** the holding device (1) is provided and designed to clamp a mobile terminal device (3) in order to be brought into different position postures, preferably by means of a swivel arm (7), and to remain in the set position posture.

4. The holding device (1) according to claim 2, **characterized in that** the holding device (1) is designed such that a screen of the electronic terminal device (3), which is provided and designed as the input/display device (6), is freely accessible to the user and/or patient during a treatment.

5. The holding device (1) according to a preceding claim, **characterized in that** the holding device (1) comprises at least one circuit (8), which is integrated in the holding device and which has a processing and charging circuit.

6. The holding device (1) according to a preceding claim, **characterized in that** the charging unit (5) is designed with integrated adapters/charging adapters, preferably USB connections, to electrically connect different models of electronic terminal devices (3) to the charging unit (5).

7. The holding device (1) according to a preceding claim, **characterized in that** the holding device (1) includes a headphone connection (10).

8. The holding device (1) according to a preceding claim, **characterized in that** the holding device (1) is provided and designed to communicate and exchange data with the connected electronic terminal device (3) via NFC and a corresponding app, preferably to directly adjust the ultrafiltration volume on the basis of an input at the electronic terminal device (3) about the ingested fluid amount of the patient.

9. The holding device (1) according to a preceding claim, **characterized in that** the holding device (1) is provided and designed to supply the mobile terminal device (3) with power via a connection (9) for a cable, preferably a supply cable, which is connected to the dialysis machine (1) or the patient bed (4), or via an energy storage unit (11), preferably a rechargeable battery.

10. A charging unit (5) comprising an integral power supply unit on a dialysis machine (2) with a holding device (1) according to one of the preceding claims.

## Revendications

1. Dispositif de maintien (1) d'une machine de dialyse (2) pour le maintien et la fixation au moins d'un terminal électronique (3), en particulier du type électronique de divertissement pendant un traitement de dialyse, dans lequel le dispositif de maintien (1) est ou peut être monté au niveau de la machine de dialyse (2) et dans lequel un dispositif de charge (5) est intégré dans le dispositif de maintien, qui est prévu et réalisé afin d'alimenter en électricité de la machine de dialyse le terminal électronique (3) maintenu et/ou fixé au niveau de la machine de dialyse, **caractérisé par**
un circuit de protection (8) qui est intégré dans le dispositif de retenue (1) afin de détecter un défaut d'un terminal (3) précisément raccordé.

2. Dispositif de maintien (1) selon la revendication 1, **caractérisé en ce que** le terminal électronique (3) et la machine de dialyse (2) sont réalisés et prévus afin de communiquer l'un avec l'autre afin de transmettre, de préférence de manière bidirectionnelle, des données entre la machine de dialyse (2) et le terminal électronique (3) au moyen d'un dispositif de saisie et d'affichage (6) du terminal électronique (3).

3. Dispositif de maintien (1) selon une revendication précédente, **caractérisé en ce que** le dispositif de maintien (1) est prévu et réalisé afin de serrer un terminal (3) mobile, afin d'être amené, de préférence au moyen d'un bras de pivotement (7), dans différents maintiens de position et de rester dans le maintien de position réglé.

4. Dispositif de maintien (1) selon la revendication 2, **caractérisé en ce que** le dispositif de maintien (1) est réalisé de telle manière qu'un écran du terminal électronique (3) qui est prévu et réalisé comme le dispositif de saisie/d'affichage (6) soit librement accessible pour l'utilisateur et/ou le patient pendant un traitement.

5. Dispositif de maintien (1) selon une revendication précédente, **caractérisé en ce que** le dispositif de maintien (1) présente au moins un circuit (8) intégré dans le dispositif de maintien, circuit qui présente un circuit de traitement et de charge.

6. Dispositif de maintien (1) selon une revendication précédente, **caractérisé en ce que** le dispositif de charge (5) est réalisé avec des adaptateurs/adaptateurs de charge intégrés, de préférence des connexions USB, afin de relier électriquement différents modèles de terminaux électroniques (3) au dispositif de charge (5).

7. Dispositif de maintien (1) selon une revendication précédente, **caractérisé en ce que** le dispositif de maintien (1) présente une prise casque (10).

8. Dispositif de maintien (1) selon une revendication précédente, **caractérisé en ce que** le dispositif de maintien (1) est prévu et réalisé afin de communiquer et d'échanger des données par NFC et une application correspondante avec le terminal électronique (3) raccordé, de préférence afin d'adapter directement le volume d'ultrafiltration sur la base d'une saisie sur le terminal électronique (3) par le biais de la quantité de liquide reçue du patient.

9. Dispositif de maintien (1) selon une revendication précédente, **caractérisé en ce que** le dispositif de maintien (1) est prévu et réalisé afin d'alimenter en électricité le terminal (3) mobile par le biais d'un raccord (9) pour un câble, de préférence un câble d'alimentation, qui est relié à la machine de dialyse (1) ou au lit du patient (4), ou par le biais d'une unité d'accumulateur d'énergie (11), de préférence une batterie.

10. Dispositif de charge (5) avec une unité d'alimentation électrique intégrale au niveau d'une machine de dialyse (2) avec un dispositif de maintien (1) selon l'une quelconque des revendications précédentes.
